# EUROPEAN PATENT SPECIFICATION

(11) **EP 1 879 915 B1**
(45) Date of publication and mention of the grant of the patent: **21.12.2016**
(21) Application number: 07715033.2
(22) Date of filing: 21.02.2007
(51) Int. Cl.: C07K 14/395

(54) **GENE ENCODING GLYCOGEN SYNTHESIS INITIATOR AND USE THEREOF**
GEN CODIEREND EINEN INITIATOR DER GLYCOGENSYNTHESE UND VERWENDUNG DAVON
GÈNE CODANT UN INITIATEUR DE SYNTHÈSE DU GLYCOGÈNE ET SON UTILISATION

(30) Priority: 01.03.2006 JP 2006055570
(43) Date of publication of application: 23.01.2008
(73) Proprietor: Suntory Holdings Limited, Kita-ku, Osaka-shi, Osaka 530-8203 (JP)
(72) Inventor: NAKAO, Yoshihiro, Mishima-gun, Osaka 6188503 (JP); KODAMA, Yukiko, Mishima-gun, Osaka 6188503 (JP); SHIMONAGA, Tomoko, Mishima-gun, Osaka 6188503 (JP)
(74) Representative: Vossius & Partner Patentanwälte Rechtsanwälte mbB
(86) International application number: PCT/JP2007/053698
(87) International publication number: WO 2007/102351

(56) References cited:
- US-B1- 6 323 001
- CHENG CHRISTINE ET AL: "Requirement of the self-glucosylating initiator proteins Glg1p and Glg2p for glycogen accumulation in Saccharomyces cerevisiae" MOLECULAR AND CELLULAR BIOLOGY, vol. 15, no. 12, 1995, pages 6632-6640, XP002437089 ISSN: 0270-7306 -& DATABASE EMBL [Online] 15 March 1996 (1996-03-15), "Saccharomyces cerevisiae Glg2p (GLG2) gene, complete cds, and Tif2p (TIF2) gene, partial cds." XP002437306 retrieved from EBI accession no. EMBL:U25436 Database accession no. U25436 -& DATABASE EMBL [Online] 21 July 1995 (1995-07-21), "Saccharomyces cerevisiae self-glucosylating initiator of glycogen synthesis (GLG1) gene, complete cds." XP002437307 retrieved from EBI accession no. EMBL:U25546 Database accession no. U25546 -& DATABASE UniProt [Online] 1 June 1994 (1994-06-01), "Glycogen synthesis initiator protein GLG1." XP002437308 retrieved from EBI accession no. UNIPROT:P36143 Databa
- ALBRECHT TANJA ET AL: "Yeast glycogenin (Glg2p) produced in Escherichia coli is simultaneously glucosylated at two vicinal tyrosine residues but results in a reduced bacterial glycogen accumulation" EUROPEAN JOURNAL OF BIOCHEMISTRY, vol. 271, no. 20, October 2004 (2004-10), pages 3978-3989, XP002437283 ISSN: 0014-2956
- DATABASE EPO Proteins [Online] 22 December 2003 (2003-12-22), "Sequence 1320 from Patent WO02068693." XP002437310 retrieved from EBI accession no. EPOP:AX929971 Database accession no. AX929971 -& DATABASE EMBL [Online] 22 December 2003 (2003-12-22), "Sequence 647 from Patent WO02068693." XP002437311 retrieved from EBI accession no. EMBL:AX929298 Database accession no. AX929298 -& WO 02/068693 A (GENOME THERAPEUTICS CORP [US]; ZENG QIANDONG [US]; KESSLER MARCO [US];) 6 September 2002 (2002-09-06)
- DATABASE EMBL [Online] 10 January 2001 (2001-01-10), "T7 end of clone AS0AA012B10 of library AS0AA from strain CLIB 533 of Saccharomyces bayanus" XP002437312 retrieved from EBI accession no. EMBL:AL398950 Database accession no. AL398950
- DATABASE EMBL [Online] 27 January 1995 (1995-01-27), "EST102504 S. cerevisiae strain X2180-1A Saccharomyces cerevisiae cDNA 3' end, mRNA sequence." XP002437313 retrieved from EBI accession no. EMBL:T37391 Database accession no. T37391
- PEREZ-TORRADO R ET AL: "Wine yeast strains engineered for glycogen overproduction display enhanced viability under glucose deprivation conditions" APPLIED AND ENVIRONMENTAL MICROBIOLOGY, vol. 68, no. 7, July 2002 (2002-07), pages 3339-3344, XP002437285 ISSN: 0099-2240
- DATABASE MEDLINE [Online] US NATIONAL LIBRARY OF MEDICINE (NLM), BETHESDA, MD, US; September 1986 (1986-09), RATNER E N ET AL: "[Characteristics of free water distribution in the cytoplasm of cryotolerant cells of Cryptococcus laurentii]" XP002437325 Database accession no. NLM3821592 & MIKROBIOLOGIIA 1986 SEP-OCT, vol. 55, no. 5, September 1986 (1986-09), pages 851-854, ISSN: 0026-3656

## Description

### TECHNICAL FIELD

The present invention relates to genes encoding glycogen synthesis initiator and use thereof, in particular, a yeast for practical use with superior resistance property to dryness and/or resistance property to low-temperature storage, alcoholic beverages produced with said yeast, and a method for producing said beverages. More particularly, the present invention relates to a yeast, whose resistance property to dryness and/or resistance property to low-temperature storage is enhanced by amplifying expression level of GLG1 or GLG2 gene encoding Glg1p or Glg2p which is a glycogen synthesis initiator in brewer's yeast, especially non-ScGLG1 gene or non-ScGLG2 gene specific to a lager brewing yeast and to a method for producing alcoholic beverages with said yeast, etc. Further, the yeast of the present invention is useful as a baker's yeast or an industrial yeast as well.

### BACKGROUND ART

Beer brewing is characterized by a process recovering yeasts after fermentation and using the recovered yeasts at the subsequent fermentation, which is called "Renjo". The yeasts are stored in the presence of ethanol in a tank whose temperature is kept at approximately 0 to 3°C. When the yeasts die during the storage, not only the next fermentation process is interfered, but also constituents of the yeast cells released by cell lysis may impart unfavorable taste to product. Therefore, it is very important for allowing some variance to design production process and for stable production of quality products to use yeasts with superior resistant property to low-temperature storage.

"Renjo" may be terminated at a certain times of fermentation is carried out. The number of times of "Renjo" may vary according to fermentation conditions or properties of yeasts used in the process. A process to develop yeasts for fermentation freshly is called propagation. Yeasts are subcultured several times enlarging scales of culture successively during the propagation process. Because propagation process requires from several days to several weeks, it brings great advantages in production efficiency if term of the process is shortened or yeast cells which are large-scale pre-cultured are able to be stored stably for extended period of time at low temperature or under dry condition.

Concerning a method for producing dry yeast maintaining high viable cell ratio, improvement of drying equipment, or improvement of manufacturing conditions such as temperature or addition of emulsifiers, etc. have been made. For example, L-drying method is not practical to be used at industrial production scale because, though it can maintain extremely high viable cell ratio, but at the same time it takes a lot of time and cost.

Regarding low-temperature resistance of yeast, some experiments designed to improve refrigeration-resistant property mainly of baker's yeast were reported. This is because Saccharomyces cerevisiae, which is a baker's yeast, has poor low-temperature storage property in comparison with brewer's yeast for beer or sake, which can ferment at low temperature. For example, baker's yeasts having refrigeration-resistant property and drying-resistant property were found out mainly by screening methods in Japanese Patent Application Laid-open No. H11-155559 and Japanese Patent Application Laid-open No. 2003-304864. Further, regarding examples utilizing genetic engineering techniques, trehalose highly accumulating strains by disruption of NTH1, which is a trehalase gene, is reported in Japanese Patent Application Laid-open No. H10-117771 and a strain highly accumulating specific amino acids such as arginine by disruption of CAR1, which is an arginase gene, is reported in Japanese Patent Application Laid-open No. 2001-238665.

Cheng, C. et al. Mol Cell Biol. (1995): 15(12), 6632-40 describes the requirement of the self-glucosylating initiator proteins Glg1p and Glg2p for glycogen accumulation in Saccharomyces cerevisiae. Albrecht T. et al. Eur J Biochem. (2004): 271(20), 3978-89 describes how yeast glycogenin (Glg2p) produced in Escherichia coli is simultaneously glucosylated at two vicinal tyrosine residues.

### DISCLOSURE OF INVENTION

Under the above situations, there has been a need to make high-efficiency production of alcoholic beverages or useful materials possible by using a gene encoding a protein responsible for drying and/or low-temperature storage-resistant property of brewery yeast and said protein.

The present inventors made extensive studies to solve the above problems and as a result, succeeded in identifying and isolating a gene encoding glycogen synthesis initiator from beer yeast. Moreover, the present inventors produced transformed yeast in which the obtained gene was expressed to verify that drying-resistant property and/or low-temperature storage-resistant property can be actually improved, thereby completing the present invention.

Thus, the present invention relates to a gene encoding a glycogen synthesis initiator of brewery yeast, to a protein encoded by said gene, to a transformed yeast in which the expression of said gene is controlled, to a method for enhancing drying-resistant property and/or low-temperature storage-resistant property of yeast using a yeast in which the expression of said gene is controlled, or the like. More specifically, the present invention provides the following polynucleotides, a vector comprising said polynucleotide, a transformed yeast introduced with said vector and the like; furthermore, a method for producing alcoholic beverages by using said transformed yeast is described.
(1) A polynucleotide selected from the group consisting of:
   (a) a polynucleotide comprising a polynucleotide consisting of the nucleotide sequence of SEQ ID NO: 1 or SEQ ID NO: 3;
   (b) a polynucleotide comprising a polynucleotide encoding a protein consisting of the amino acid sequence of SEQ ID NO: 2 or SEQ ID NO: 4;
   (c) a polynucleotide comprising a polynucleotide encoding a protein consisting of the amino acid sequence of SEQ ID NO: 2 or SEQ ID NO: 4 in which one to 10 amino acids thereof are deleted, substituted, inserted and/or added, and having a glycogen synthesis initiation activity; and
   (d) a polynucleotide comprising a polynucleotide encoding a protein having an amino acid sequence having 90% or higher identity with the amino acid sequence of SEQ ID NO: 2 or SEQ ID NO: 4, and said protein having a glycogen synthesis initiation activity.
(2) The polynucleotide according to (1) above selected from the group consisting of:
   (g) a polynucleotide comprising a polynucleotide encoding a protein consisting of the amino acid sequence of SEQ ID NO: 2 or SEQ ID NO: 4, or encoding the amino acid sequence of SEQ ID NO: 2 or SEQ ID NO: 4 in which 1 to 2 amino acids thereof are deleted, substituted, inserted, and/or added, and wherein said protein has a glycogen synthesis initiation activity; and
   (h) a polynucleotide comprising a polynucleotide encoding a protein having 98% or higher identity with the amino acid sequence of SEQ ID NO: 2 or SEQ ID NO: 4, and having a glycogen synthesis initiation activity.
(3) The polynucleotide according to (1) above comprising a polynucleotide consisting of the nucleotide sequence of SEQ ID NO: 1 or SEQ ID NO: 3.
(4) The polynucleotide according to (1) above comprising a polynucleotide encoding a protein consisting of the amino acid sequence of SEQ ID NO: 2 or SEQ ID NO: 4.
(5) The polynucleotide according to any one of (1) to (4) above, wherein the polynucleotide is DNA.
(6) A protein encoded by the polynucleotide according to any one of (1) to (5) above.
(7) A vector containing the polynucleotide according to any one of (1) to (5) above.

Further disclosed are:
(7a) The vector of (7) above, which comprises the expression cassette comprising the following components:
   (x) a promoter that can be transcribed in a yeast cell;
   (y) any of the polynucleotides described in (1) to (5) above linked to the promoter in a sense or antisense direction; and
   (z) a signal that can function in a yeast with respect to transcription termination and polyadenylation of a RNA molecule.
(7b) The vector of (7) above, which comprises the expression cassette comprising the following components:
   (x) a promoter that can be transcribed in a yeast cell;
   (y) any of the polynucleotides described in (1) to (5) above linked to the promoter in a sense direction; and
   (z) a signal that can function in a yeast with respect to transcription termination and polyadenylation of a RNA molecule.

The present invention provides:
(8) A yeast into which the vector according to any one of (7) to (7b) above has been introduced.
(9) The yeast (yeast for practical use) according to (8) above, wherein drying-resistant property is increased. The "yeast for practical use" means that a yeast which possesses practical value such as brewer's (brewery) yeast, baker's yeast or industrial yeast, etc.
(10) The yeast according to (8) above, wherein low-temperature storage-resistant property is increased.
(11) The yeast according to (9) above, wherein the drying-resistant property is increased by increasing an expression level of the protein of (6) above.
(12) The yeast according to (10) above, wherein the low-temperature storage-resistant property is increased by increasing an expression level of the protein of (6) above.
   (12a) The yeast according to any one of (9) to (12) above, wherein the yeast is a brewery yeast.
(13) A method for producing an alcoholic beverage by using the yeast according to any one of (8) to (12a) above.
(14) The method according to (13) above, wherein the brewed alcoholic beverage is a malt beverage.
(15) The method according to (13) above, wherein the brewed alcoholic beverage is wine.

Further disclosed is:
(16) An alcoholic beverage produced by the method according to any one of (13) to (15) above.

The invention provides:
(17) A method for assessing a test yeast for its drying-resistant property and/or low-temperature storage-resistant property, comprising using a primer or probe designed based on the nucleotide sequence of a gene having the nucleotide sequence of SEQ ID NO:1 or SEQ ID NO: 3 and encoding a glycogen synthesis initiator. Further disclosed are:
   (17a) A method for selecting a yeast having an increased drying-resistant property and/or low-temperature storage-resistant property by using the method described in (17) above.
   (17b) A method for producing an alcoholic beverage (for example, beer or alcohol for industrial use, etc.) by using the yeast selected with the method described in (17a) above.
   (17c) A method for producing an useful materials (for example, protein) by using the yeast selected with the method described in (17a) above. The invention provides:
(18) A method for assessing a test yeast for its drying-resistant property and/or low-temperature storage-resistant property, comprising: culturing the test yeast; and measuring the expression level of the gene having the nucleotide sequence of SEQ ID NO: 1 or SEQ ID NO: 3 and encoding a glycogen synthesis initiator. Further disclosed are:
   (18a) A method for selecting a yeast having a high drying-resistant property and/or low-temperature storage-resistant property, which comprises assessing a test yeast by the method described in (18) above and selecting a yeast having a high expression level of gene encoding a glycogen synthesis initiator.
   (18b) A method for producing an alcoholic beverage (for example, beer) by using the yeast selected with the method described in (18a) above. The invention provides:
   (18c) A method for producing an useful material (for example, protein) by using the yeast selected with the method described in (18a) above.
(19) A method for selecting a yeast, comprising: culturing test yeasts; quantifying the protein of (6) above or measuring the expression level of the gene having the nucleotide sequence of SEQ ID NO: 1 or SEQ ID NO: 3 and encoding a glycogen synthesis initiator; and selecting a test yeast having an amount of the protein or the gene expression level according to favorable drying-resistant property and/or low-temperature storage-resistant property.
(20) The method for selecting a yeast according to (19) above, comprising: culturing a reference yeast and test yeasts; measuring for each yeast the expression level of the gene having the nucleotide sequence of SEQ ID NO: 1 or SEQ ID NO: 3 and encoding a glycogen synthesis initiator; and selecting a test yeast having the gene expression higher than that in the reference yeast.
(21) The method for selecting a yeast according to (19) above, comprising: culturing a reference yeast and test yeasts; quantifying the protein according to (6) above in each yeast; and selecting a test yeast having a larger amount of the protein than that in the reference yeast. The invention provid
(22) A method for producing an alcoholic beverage comprising: conducting fermentation using the yeast according to any one of (8) to (12a) above or a yeast selected by the methods according to any one of (19) to (21) above.

The transformed yeast of the present invention is able to keep high viable cell ratio during dry storage or low-temperature storage. Therefore, when it is used for brewing and so on, painfulness of conserving yeast can be eliminated. Further, it is expected to contribute to quality stabilization. Moreover, dry yeast is suitable for long-storage, and it is very advantageous to distribution or transportation due to its reduced weight. It is also useful as microorganisms for industrial application such as industrial alcohol production or production of useful proteins. The yeast of the present invention also useful as a baker's yeast or an industrial yeast as well.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test. The horizontal axis represents fermentation time while the vertical axis represents apparent extract concentration (w/w%).
Figure 3 shows the expression profile of non-ScGLG1 gene in yeasts upon beer fermentation test. The horizontal axis represents fermentation time while the vertical axis represents the intensity of detected signal.
Figure 4 shows the result of drying-resistant property test of parent strain and non-ScGLG1 highly expressed strain.
Figure 5 shows the result of low-temperature storage-resistant property test of parent strain and non-ScGLG1 highly expressed strain.
Figure 6 shows the cell growth with time upon beer fermentation test. The horizontal axis represents fermentation time while the vertical axis represents optical density at 660 nm (OD660).
Figure 7 shows the extract (sugar) consumption with time upon beer fermentation test. The horizontal axis represents fermentation time while the vertical axis represents apparent extract concentration (w/w%).
Figure 8 shows the expression profile of non-ScGLG2 gene in yeasts upon beer fermentation test. The horizontal axis represents fermentation time while the vertical axis represents the intensity of detected signal.
Figure 9 shows the result of drying-resistant property test of parent strain and non-ScGLG2 highly expressed strain.
Figure 10 shows the result of low-temperature storage-resistant property test of parent strain and non-ScGLG2 highly expressed strain.

### BEST MODES FOR CARRYING OUT THE INVENTION

The present inventors isolated and identified non-ScGLG1 gene and non-ScGLG2 gene encoding glycogen synthesis initiators of brewery yeast based on the lager brewing yeast genome information mapped according to the method disclosed in Japanese Patent Application Laid-Open No. 2004-283169. The nucleotide sequences of the genes are represented by SEQ ID NO: 1 and SEQ ID NO: 3, respectively. Further, amino acid sequences of proteins encoded by the genes are represented by SEQ ID NO: 2 and SEQ ID NO: 4, respectively.

### 1. Polynucleotide of the invention

First of all, the present invention provides (a) a polynucleotide comprising a polynucleotide of the nucleotide sequence of SEQ ID NO: 1 or SEQ ID NO: 3; and (b) a polynucleotide comprising a polynucleotide encoding a protein of the amino acid sequence of SEQ ID NO: 2 or SEQ ID NO: 4. The polynucleotide can be DNA or RNA.

The target polynucleotide of the present invention is not limited to the polynucleotide encoding a protein having a glycogen synthesis initiation activity described above and may include other polynucleotides encoding proteins having equivalent functions to said protein. Proteins with equivalent functions include, for example, (c) a protein of an amino acid sequence of SEQ ID NO: 2 or SEQ ID NO: 4 with one or more amino acids thereof being deleted, substituted, inserted and/or added and having a glycogen synthesis initiation activity.

Such proteins include a protein consisting of an amino acid sequence of SEQ ID NO: 2 or SEQ ID NO: 4 with, for example,

1 to 10, 1 to 9, 1 to 8, 1 to 7, 1 to 6 (1 to several amino acids), 1 to 5, 1 to 4, 1 to 3, 1 to 2, or 1 amino acid residues thereof being deleted, substituted, inserted and/or added and having a glycogen synthesis initiation activity. In general, the number of deletions, substitutions, insertions, and/or additions is preferably smaller. In addition, such proteins include (d) a protein having an amino acid sequence with 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2 or SEQ ID NO: 4, and having a glycogen synthesis initiation activity. In general, the percentage identity is preferably higher.

Glycogen synthesis initiation activity may be evaluated by measuring glycogen content in yeast cells, for example, by a method described in Cheng et al., Mol. Cell. Biol., 15(12), 6632-6640 (1995).

Furthermore, the present disclosure also contemplates (e) a polynucleotide comprising a polynucleotide which hybridizes to a polynucleotide consisting of a nucleotide sequence complementary to the nucleotide sequence of SEQ ID NO: 1 or SEQ ID NO: 3 under stringent conditions and which encodes a protein having a glycogen synthesis initiation activity; and (f) a polynucleotide comprising a polynucleotide which hybridizes to a polynucleotide complementary to a nucleotide sequence of encoding a protein of SEQ ID NO: 2 or SEQ ID NO: 4 under stringent conditions, and which encodes a protein having a glycogen synthesis initiation activity.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO: 1 or SEQ ID NO: 3 or polynucleotide encoding the amino acid sequence of SEQ ID NO: 2 or SEQ ID NO: 4 as a probe. The hybridization method may be a method described, for example, in MOLECULAR CLONING 3rd Ed., CURRENT PROTOCOLS IN MOLECULAR BIOLOGY, John Wiley & Sons 1987-1997, and so on.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO: 2 or SEQ ID NO: 4 as calculated by homology search software, such as FASTA and BLAST using default parameters.

Identity between amino acid sequences or nucleotide sequences may be determined using algorithm BLAST by Karlin and Altschul (Proc. Natl. Acad. Sci. USA, 87: 2264-2268, 1990; Proc. Natl. Acad. Sci. USA, 90: 5873, 1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score =100 and word length =12. When an amino acid sequence is sequenced using BLASTX, the parameters are, for example, score = 50 and word length = 3. When BLAST and Gapped BLAST programs are used, default parameters for each of the programs are employed.

### 2. Protein of the present invention

The present disclosure also provides proteins encoded by any of the polynucleotides (a) to (i) above. A preferred protein of the present invention comprises an amino acid sequence of SEQ ID NO: 2 or SEQ ID NO: 4 with one or several amino acids thereof being deleted, substituted, inserted and/or added, and having a glycogen synthesis initiation activity.

Such protein includes those having an amino acid sequence of SEQ ID NO: 2 or SEQ ID NO: 4 with amino acid residues thereof of the number mentioned above being deleted, substituted, inserted and/or added and having a glycogen synthesis initiation activity. In addition, such protein includes those having homology as described above with the amino acid sequence of SEQ ID NO: 2 or SEQ ID NO: 4 and having a glycogen synthesis initiation activity.

Such proteins may be obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl. Acad Sci. USA 79: 6409 (1982), Gene 34: 315 (1985), Nuc. Acids Res., 13: 4431 (1985), Proc. Natl. Acad. Sci. USA 82: 488 (1985).

Deletion, substitution, insertion and/or addition of one or more amino acid residues in an amino acid sequence of the protein of the invention means that one or more amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Grou D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and yeast transformed with the vector

The present invention then provides a vector comprising the polynucleotide described above. The vector of the present disclosure is directed to a vector including any of the polynucleotides described in (a) to (i) above or any of the polynucleotides described in (j) to (m) above. Generally, the vector of the present disclosure comprises an expression cassette including as components (x) a promoter that can transcribe in a yeast cell; (y) a polynucleotide described in any of (a) to (i) above that is linked to the promoter in sense or antisense direction; and (z) a signal that functions in the yeast with respect to transcription termination and polyadenylation of RNA molecule. Further, in order to highly express the protein of the invention, these polynucleotides are preferably introduced in the sense direction to the promoter to promote expression of the polynucleotide (DNA) described in any of (a) to (i) above.

A vector introduced in the yeast may be any of a multicopy type (YEp type), a single copy type (YCp type), or a chromosome integration type (YIp type). For example, YEp24 (J. R Broach et al., EXPERIMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the yeast for practical use and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a yeast for practical use, for example, a geneticin-resistant gene (G418r), a copper-resistant gene (CUP1) (Marin et al., Proc. Natl. Acad. Sci. USA, 81, 337 1984) or a cerulenin-resistant gene (fas2m, PDR4) (Junji Inokoshi et al., Biochemistry, 64, 660, 1992; and Hussain et al., Gene, 101: 149, 1991, respectively) may be used.

A vector constructed as described above is introduced into a host yeast. Examples of the host yeast include any yeast (yeast for practical use) that can be used for brewing, for example, brewery yeasts for beer, wine and sake, baker's yeast, yeast for producing industrial alcohol or yeast for producing useful proteins and so on. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan, baker's yeast such as NBRC0555, NBRC1346 or NBRC2043, etc., may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth. Enzym., 194: 182 (1990)), a spheroplast method (Proc. Natl. Acad Sci. USA, 75: 1929(1978)), a lithium acetate method (J. Bacteriology, 153: 163 (1983)), and methods described *in* Proc. Natl. Acad. Sci. USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M. After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY)

### 4. Method of producing alcoholic beverages according to the present invention and alcoholic beverages produced by the method

A yeast having a superior drying-resistant property and/or low-temperature storage-resistant property can be obtained by introducing the vector of the present invention described above to a yeast. Further, a yeast having a superior drying-resistant property and/or low-temperature storage-resistant property can be obtained by selecting a yeast by the yeast assessment method of the present invention described below. The target use of yeasts obtained in the present invention include, for example, but not limited to, brewing alcoholic beverages such as beer, wine, whisky, sake and the like, baking bread, manufacturing useful materials such as industrial alcohol production and production of useful proteins.

In order to produce these products, a known technique can be used except that a yeast for practical use obtained according to the present invention is used in the place of a parent strain. Since starting materials, manufacturing equipment, manufacturing control and the like may be the same as the conventional ones; it can be performed without increasing cost.

### 5. Yeast assessment method of the invention

The present invention relates to a method for assessing a test yeast for its drying-resistant property and/or low-temperature storage-resistant property by using a primer or a probe designed based on a nucleotide sequence of a gene having the nucleotide sequence of SEQ ID NO: 1 or SEQ ID NO: 3 and encoding a glycogen synthesis initiator. General technique for such assessment method is known and is described in, for example, WO01/040514, Japanese Laid-Open Patent Application No. H8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the gene encoding a glycogen synthesis initiator, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the drying-resistant property and/or low-temperature storage-resistant property of yeast as determined by whether the molecular weight of the amplified product is a size that contains the DNA molecule of the specific part. In addition, by analyzing the nucleotide sequence of the amplified product, the property may be predicted and/or assessed more precisely.

Moreover, in the present invention, a test yeast is cultured to measure an expression level of the gene encoding a glycogen synthesis initiator having the nucleotide sequence of SEQ ID NO: 1 or SEQ ID NO: 3 to assess the test yeast for its drying-resistant property and/or low-temperature storage-resistant property. Measurement of expression level of the gene encoding a glycogen synthesis initiator can be performed by culturing test yeast and then quantifying mRNA or a protein resulting from the gene. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the gene encoding a glycogen synthesis initiator having the nucleotide sequence of SEQ ID NO: 1 or SEQ ID NO: 3 are measured to select a test yeast with the gene expression level according to the target glycogen-producing ability, thereby a yeast favorable for brewing desired alcoholic beverages can be selected. In addition, a reference yeast and a test yeast may be cultured so as to measure and compare the expression level of the gene in each of the yeasts, thereby a favorable test yeast can be selected. More specifically, for example, a reference yeast and one or more test yeasts are cultured and an expression level of the gene encoding a glycogen synthesis initiator having the nucleotide sequence of SEQ ID NO: 1 or SEQ ID NO: 3 is measured in each yeast. By selecting a test yeast with the gene expressed higher than that in the reference yeast, a yeast suitable for brewing desired alcoholic beverages or production ofuseful materials can be selected.

Alternatively, test yeasts are cultured and a yeast with a high glycogen synthesis initiation activity is selected, thereby a yeast suitable for brewing desired alcoholic beverages or production of useful materials can be selected.

In these cases, the test yeasts or the reference yeast may be, for example, a yeast introduced with the vector of the invention, an artificially mutated yeast or a naturally mutated yeast. The glycogen synthesis initiation activity can be evaluated by measuring glycogen content in yeast cells by, for example, a method described in Cheng et al., Mol. Cell. Biol., 15(12), 6632-6640 (1995). The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see, e.g.,* Yasuji Oshima Ed., BIOCHEMISTRY EXPERIMENTS vol. 39, Yeast Molecular Genetic Experiments, pp. 67-75, JSSP).

In addition, examples of yeasts used as the reference yeast or the test yeasts include any yeasts (yeasts for practical use), for example, brewery yeasts for beer, wine, sake and the like or baker's yeast, yeast for producing industrial alcohol or yeast for producing useful proteins, etc. More specifically, yeasts such as genus *Saccharomyces* may be used (*e.g., S. pastorianus, S. cerevisiae,* and *S. carlsbergensis*). According to the present invention, a lager brewing yeast, for example, *Saccharomyces pastorianus* W34/70; *Saccharomyces carlsbergensis* NCYC453 or NCYC456; or *Saccharonayces cerevisiae* NBRC1951, NBRC1952, NBRC1953 or NBRC1954, etc., may be used. Further, wine yeasts such as wine yeasts #1, 3 and 4 from the Brewing Society of Japan; and sake yeasts such as sake yeast #7 and 9 from the Brewing Society of Japan, baker's yeast such as NBRC0555, NBRC1346 and NBRC2043, etc., may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of Gene Encoding Glycogen synthesis initiator (non-SeGLG1)

A gene encoding a glycogen synthesis initiator of lager brewing yeast (non-ScGLG1) (SEQ ID NO: 1) was found as a result of a search utilizing the comparison database described in Japanese Patent Application Laid-Open No. 2004-283169. Based on the acquired nucleotide sequence information, primers non-ScGLG1_for (SEQ ID NO: 5) and non-ScGLG1_rv (SEQ ID NO: 6) were designed to amplify the full-length of the gene. PCR was carried out using chromosomal DNA of a genome sequencing strain, Saccharomyces pastorianus Weihenstephan 34/70 (sometimes abbreviated as "W34/70 strain"), as a template to obtain DNA fragments including the full-length gene of non-ScGLG1.

The non-ScGLG1 gene fragments thus obtained were inserted into pCR2.1-TOPO vector (Invitrogen) by TA cloning. The nucleotide sequences of the non-ScGLG1 gene were analyzed by Sanger's method (F. Sanger, Science, 214: 1215, 1981) to confirm the nucleotide sequence.

### Example 2: Analysis of Expression of non-ScGLG1 Gene during Beer Fermentation

A beer fermentation test was conducted using a lager brewing yeast, Saccharomyces pastorianus W34/70, and mRNA extracted from the lager brewing yeast during fermentation was detected by a beer yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

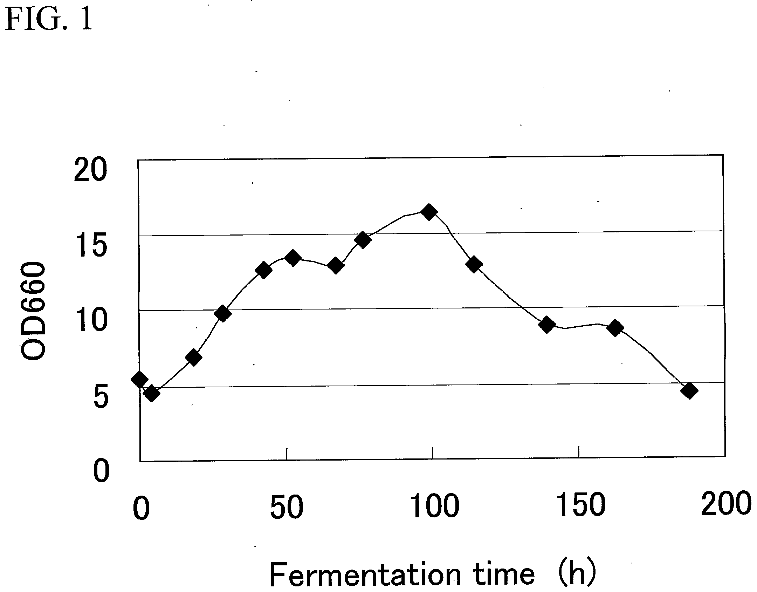

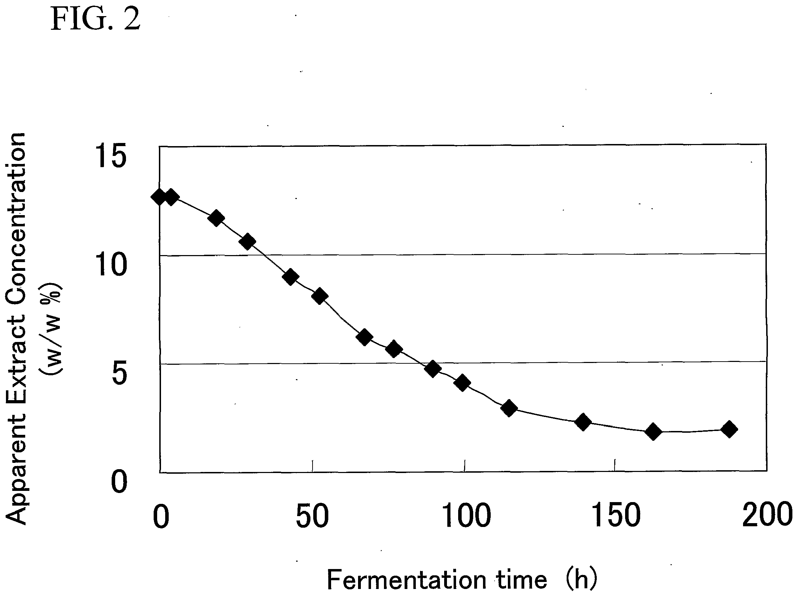

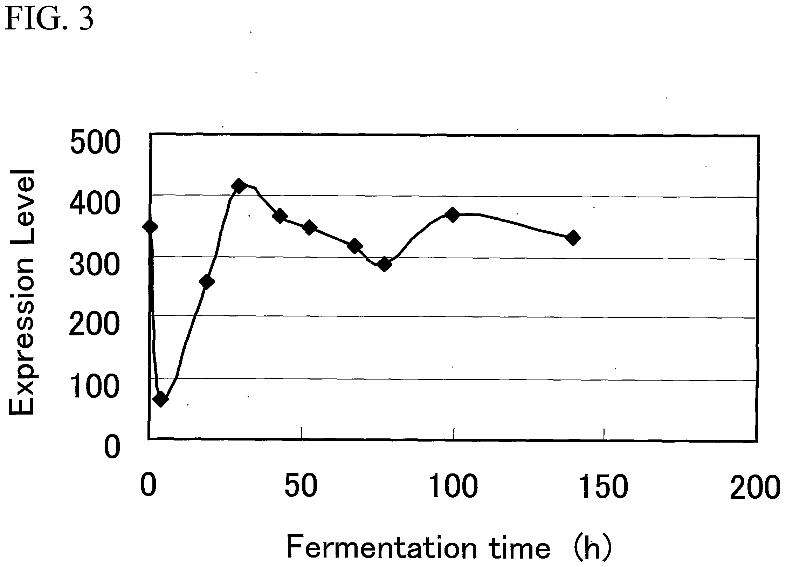

The fermentation liquor was sampled over time, and the time-course changes in amount of yeast cell growth (Fig. 1) and apparent extract concentration (Fig. 2) were observed. Simultaneously, yeast cells were sampled to prepare mRNA, and the prepared mRNA was labeled with biotin and was hybridized to a beer yeast DNA microarray. The signal was detected using GeneChip Operating system (GCOS; GeneChip Operating Software 1.0, manufactured by Affymetrix Co). Expression pattern of the non-ScGLG1 gene is shown in Figure 3. This result confirmed the expression of the non-ScGLG1 gene in the general beer fermentation.

### Example 3: Construction of non-ScGLG1 Highly Expressed Strain

The non-ScGLG1/pCR2.1-TOPO described in Example 1 was digested with the restriction enzymes SacI and NotI to prepare a DNA fragment containing the entire length of the protein-encoding region. This fragment was ligated to pYCGPYNot treated with the restriction enzymes SacI and NotI, thereby constructing the non-ScGLG1 high expression vector non-ScGLG1/pYCGPYNot. pYCGPYNot is a YCp-type yeast expression vector. A gene inserted is highly expressed by the pyruvate kinase gene PYK1 promoter. The geneticin-resistant gene G418^{r} is included as the selectable marker in the yeast, and the ampicillin-resistant gene Amp^{r} as the selectable marker in Escherichia coli.

Using the high expression vector prepared by the above method, an AJL4004 strain was transformed by the method described in Japanese Patent Application Laid-open No. H07-303475. The transformants were selected on a YPD plate medium (1% yeast extract, 2% polypeptone, 2% glucose and 2% agar) containing 300 mg/L of geneticin.

### Example 4: Evaluation of Drying-resistant Property of non-ScGLG1 Highly Expressed Strain

Drying-resistant properties of the parent strain (AJL4004 strain) and the non-ScGLG1 highly expressed strain obtained by the method described in Example 3 were evaluated by a method described below.

One platinum loopful of each yeast was inoculated into 10 mL of wort containing 100 mg/L of geneticin, and stirred at 30°C overnight (precultivation). The precultivation liquid was inoculated into 10 mL wort containing 100 mg/L of geneticin to make its OD660 = 0.5, then main culture was initiated. The culture was continued for 2 days until the growth of the yeast reached stationary phase. Turbidity of the culture was measured at the completion of the culture, then the culture liquid was suspended in sterile water to make its OD = 2. One hundred microliter (100 µL) of the suspension thus obtained was dispensed into a 1.5 mL microtube, then the yeast cells were dried by evaporation for 1 hour using a reduced-pressure concentrator (DNA110 SpeedVac (registered trademark), manufactured by ThermoSavant).

Viable cell ratio was measured by a method described below. The dried yeast cells obtained above were resuspended in 50 µL of sterile water, then 50 µL of 0.02% methylene blue solution (pH 4.5) was added to the suspension. Blue-stained yeast cells which had lost reducing power were considered as dead yeast cells. Then the suspension was observed under a microscope, and viable cell ratio was measured using a Cell Vital Analyzer System (DA cell counter, manufactured by Yamato Scientific Co., Ltd.). The cells were counted until the population reached more than 2000 cells to minimize experimental error.

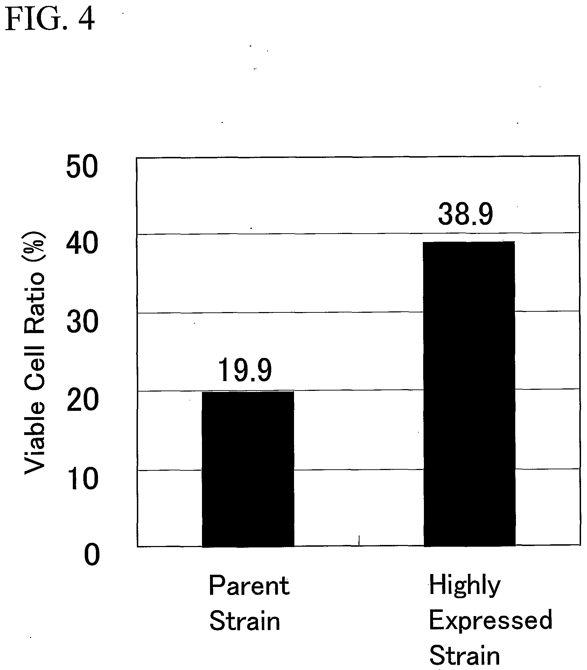

As indicated in Figure 4, viable cell ratio of the highly-expressed strain was 38.9%, though viable cell ratio of the parent strain was 19.9%. It was demonstrated by the results that drying-resistant property of yeast was increased by high expression of non-ScGLG1.

### Example 5: Evaluation of Low-temperature Resistant Property of non-ScGLG1 Highly Expressed Strain

Low-temperature resistant property of the parent strain (AJL4004 strain) and the non-ScGLG1 highly expressed strain obtained by the method described in Example 3 are evaluated by the mehtod described below. Nine hundred microliter (900 µL) of the yeast suspensions cultured by the method described in Example 4 and prepared as OD660=2 are dispensed into two microtubes, respectively. One hundred microliter (100 µL) of sterile water is added to one of the microtubes, on the other hand, 100 µL of 99.5 % ethanol is added to another one (final concentration is 10%). The suspensions are stored at 5°C for 4 weeks, then viable cell ratios are measured by the same method as Example 4.

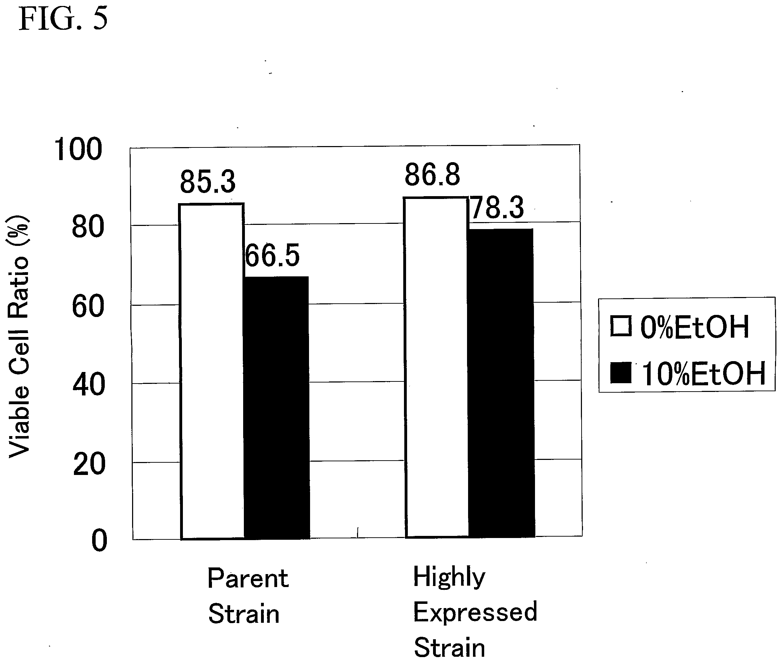

As indicated in Figure 5, viable cell ratio of non-ScGLG1 highly expressed strain after storage was higher than that of the parent strain. The effect was more obvious under 10% ethanol. It was demonstrated by the results that low-temperature storage-resistant property of yeast was increased by high expression of non-ScGLG1.

### Example 6: Cloning of Gene Encoding Glycogen synthesis initiator (non-ScGLG2)

A gene encoding a glycogen synthesis initiator of lager brewing yeast (non-ScGLG2) (SEQ ID NO: 3) was found as a result of a search utilizing the comparison database described in Japanese Patent Application Laid-Open No. 2004-283169. Based on the acquired nucleotide sequence information, primers non-ScGLG2_for (SEQ ID NO: 7) and non-ScGLG2_rv (SEQ ID NO: 8) were designed to amplify the full-length of the gene. PCR was carried out using chromosomal DNA of a genome sequencing strain, Saccharomyces pastorianus Weihenstephan 34/70, as a template to obtain DNA fragments including the full-length gene of non-ScGLG2.

The non-ScGLG2 gene fragments thus obtained were inserted into pCR2.1-TOPO vector (Invitrogen) by TA cloning. The nucleotide sequences of the non-ScGLG2 gene were analyzed by Sanger's method (F. Sanger, Science, 214: 1215, 1981) to confirm the nucleotide sequence.

### Example 7: Analysis of Expression of non-ScGLG2 Gene during Beer Fermentation

A beer fermentation test was conducted using a lager brewing yeast, Saccharomyces pastorianus W34/70, and mRNA extracted from the lager brewing yeast during fermentation was detected by a beer yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

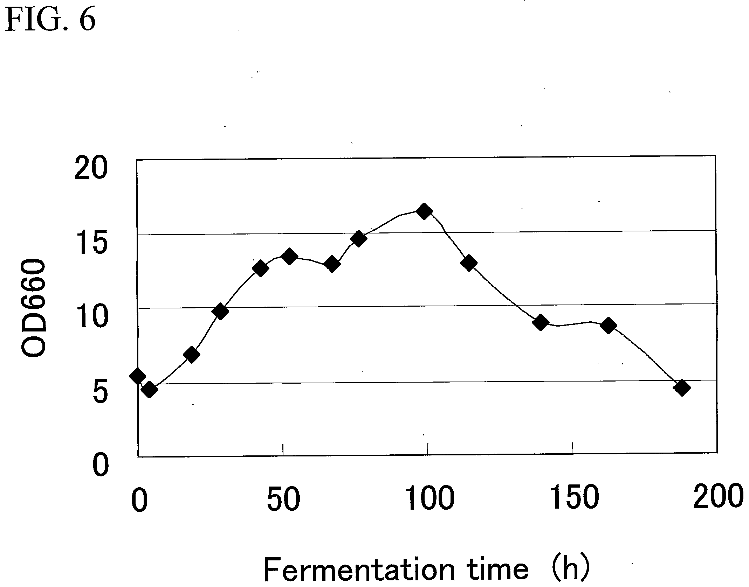

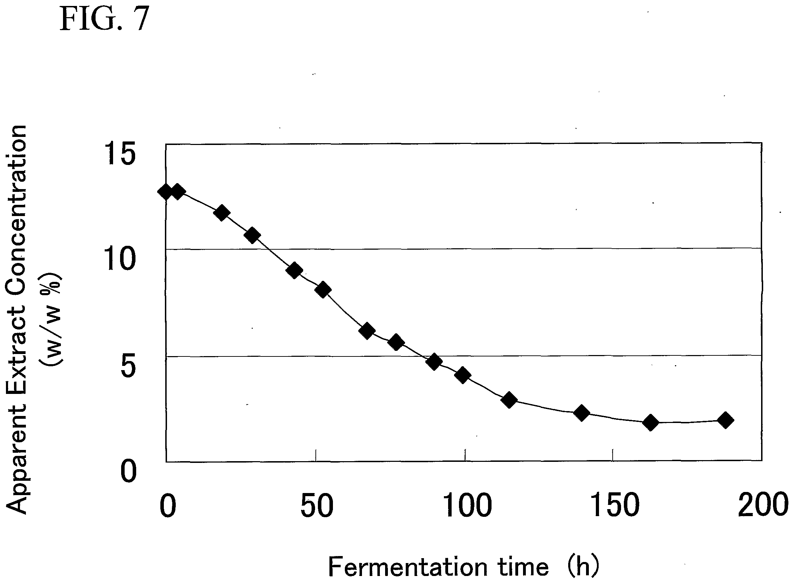

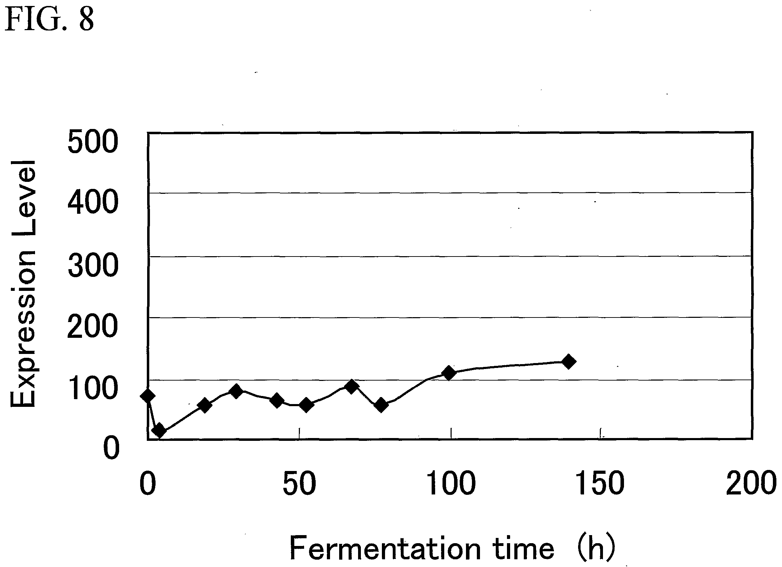

The fermentation liquor was sampled over time, and the time-course changes in amount of yeast cell growth (Fig. 6) and apparent extract concentration (Fig. 7) were observed. Simultaneously, yeast cells were sampled to prepare mRNA, and the prepared mRNA was labeled with biotin and was hybridized to a beer yeast DNA microarray. The signal was detected using GeneChip Operating system (GCOS; GeneChip Operating Software 1.0, manufactured by Affymetrix Co). Expression pattern of the non-ScGLG2 gene is shown in Figure 8. This result confirmed the expression of the non-ScGLG2 gene in the general beer fermentation.

### Example 8: Construction of non-ScGLG2 Highly Expressed Strain

The non-ScGLG2/pCR2.1-TOPO described in Example 6 was digested with the restriction enzymes SacI and NotI to prepare a DNA fragment containing the entire length of the protein-encoding region. This fragment was ligated to pYCGPYNot treated with the restriction enzymes SacI and NotI, thereby constructing the non-ScGLG2 high expression vector non-ScGLG2/pYCGPYNot.

Using the high expression vector prepared by the above method, an AJL4004 strain was transformed by the method described in Japanese Patent Application Laid-open No. H07-303475. The transformants were selected on a YPD plate medium (1% yeast extract, 2% polypeptone, 2% glucose and 2% agar) containing 300 mg/L of geneticin.

### Example 9: Evaluation of Drying-resistant Property of non-ScGLG2 Highly Expressed Strain

Drying-resistant properties of the parent strain (AJL4004 strain) and the non-ScGLG2 highly expressed strain obtained by the method described in Example 8 were evaluated by the same method as Example 4.

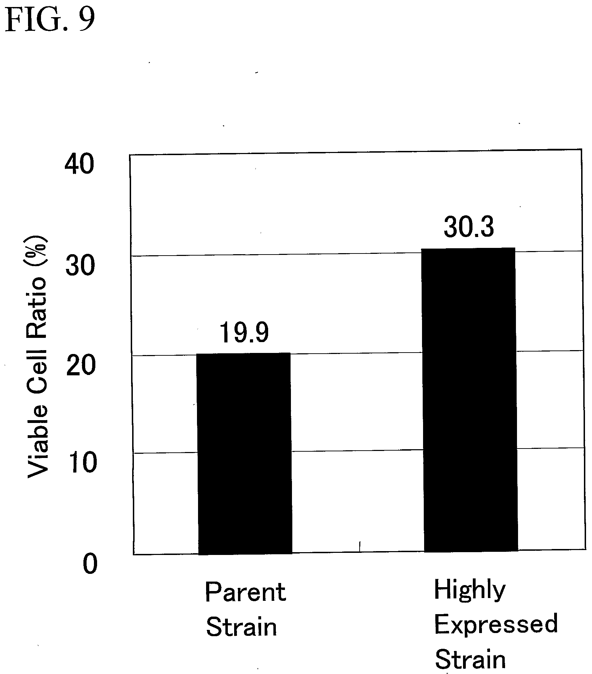

As indicated in Figure 9, viable cell ratio of the highly-expressed strain was 30.3%, though viable cell ratio of the parent strain was 19.9%. It was demonstrated by the results that drying-resistant property of yeast was increased by high expression of non-ScGLG2.

### Example 10: Evaluation of Low-temperature Resistant Property of non-ScGLG2 Highly Expressed Strain

Low-temperature resistant property of the parent strain (AJL4004 strain) and the non-ScGLG2 highly expressed strain obtained by the method described in Example 8 were evaluated by the same method as Example 5.

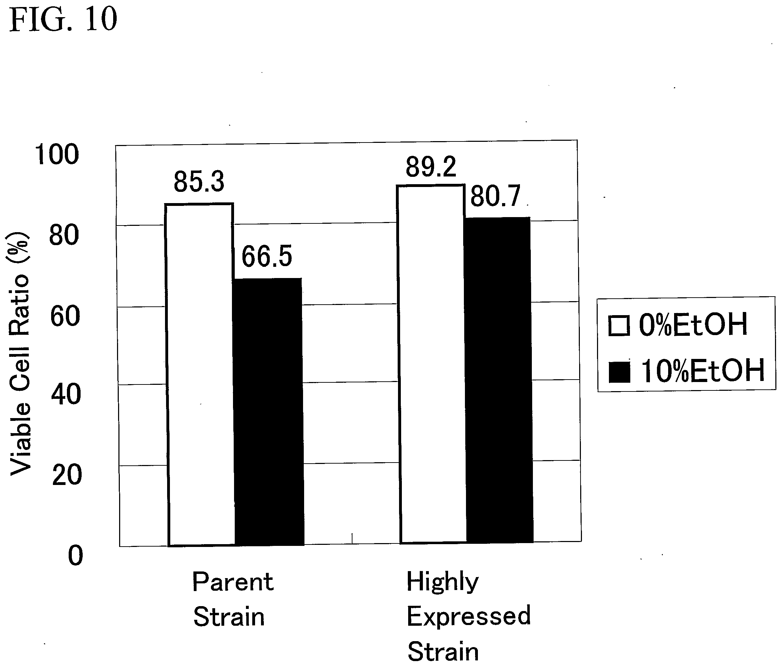

As indicated in Figure 10, viable cell ratio of non-ScGLG2 highly expressed strain after storage was higher than that of the parent strain. The effect was more obvious under 10% ethanol. It was demonstrated by the results that low-temperature storage-resistant property of yeast was increased by high expression of non-ScGLG2.

### INDUSTRIAL APPLICABILITY

According to the present invention, yeast can be stored stably for extended period of time, because drying-resistant property and/or low-temperature storage-resistant property can be enhanced by the present invention. Accordingly, efficiency of brewing alcoholic beverages (such as beer), production of bread, or manufacturing useful materials such as industrial alcohol production or production of useful proteins, etc., can be improved by the present invention.
## Claims

1. A polynucleotide selected from the group consisting of:
(a) a polynucleotide comprising a polynucleotide consisting of the nucleotide sequence of SEQ ID NO: 1 or SEQ ID NO: 3;
(b) a polynucleotide comprising a polynucleotide encoding a protein consisting of the amino acid sequence of SEQ ID NO: 2 or SEQ ID NO: 4;
(c) a polynucleotide comprising a polynucleotide encoding a protein consisting of the amino acid sequence of SEQ ID NO: 2 or SEQ ID NO: 4 in which one to 10 amino acids thereof are deleted, substituted, inserted and/or added, and having a glycogen synthesis initiation activity; and
(d) a polynucleotide comprising a polynucleotide encoding a protein having an amino acid sequence having 90% or higher identity with the amino acid sequence of SEQ ID NO: 2 or SEQ ID NO: 4, and said protein having a glycogen synthesis initiation activity.

2. The polynucleotide according to Claim 1 selected from the group consisting of:
(g) a polynucleotide comprising a polynucleotide encoding a protein consisting of the amino acid sequence of SEQ ID NO: 2 or SEQ ID NO: 4, or encoding the amino acid sequence of SEQ ID NO: 2 or SEQ ID NO: 4 in which 1 to 2 amino acids thereof are deleted, substituted, inserted, and/or added, and wherein said protein has a glycogen synthesis initiation activity; and
(h) a polynucleotide comprising a polynucleotide encoding a protein having 98% or higher identity with the amino acid sequence of SEQ ID NO: 2 or SEQ ID NO: 4, and having a glycogen synthesis initiation activity.

3. The polynucleotide according to Claim 1 comprising a polynucleotide consisting of the nucleotide sequence of SEQ ID NO: 1 or SEQ ID NO: 3.

4. The polynucleotide according to Claim 1 comprising a polynucleotide encoding a protein consisting of the amino acid sequence of SEQ ID NO: 2 or SEQ ID NO: 4.

5. The polynucleotide according to any one of Claims 1 to 4, wherein the polynucleotide is DNA.

6. A protein encoded by the polynucleotide according to any one of Claims 1 to 5.

7. A vector containing the polynucleotide according to any one of Claims 1 to 5.

8. A yeast into which the vector according to Claim 7 has been introduced.

9. The yeast according to Claim 8, wherein drying-resistant property is increased.

10. The yeast according to Claim 8, wherein low-temperature storage-resistant property is increased.

11. The yeast according to Claim 9, wherein the drying-resistant property is increased by increasing an expression level of the protein of Claim 6.

12. The yeast according to Claim 10, wherein the low-temperature storage-resistant property is increased by increasing an expression level of the protein of Claim 6.

13. A method for producing an alcoholic beverage by using the yeast according to any one of Claims 8 to 12.

14. The method according to Claim 13, wherein the brewed alcoholic beverage is a malt beverage.

15. The method according to Claim 13, wherein the brewed alcoholic beverage is wine.

16. A method for assessing a test yeast for its drying-resistant property and/or low-temperature storage-resistant property, comprising using a primer or probe designed based on the nucleotide sequence of a gene having the nucleotide sequence of SEQ ID NO: 1 or SEQ ID NO: 3 and encoding a glycogen synthesis initiator.

17. A method for assessing a test yeast for its drying-resistant property and/or low-temperature storage-resistant property, comprising: culturing the test yeast; and measuring the expression level of the gene having the nucleotide sequence of SEQ ID NO: 1 or SEQ ID NO: 3 and encoding a glycogen synthesis initiator.

18. A method for selecting a yeast, comprising: culturing test yeasts; quantifying the protein of Claim 6 or measuring the expression level of the gene having the nucleotide sequence of SEQ ID NO: 1 or SEQ ID NO: 3 and encoding a glycogen synthesis initiator; and selecting a test yeast having an amount of the protein or the gene expression level according to favorable drying-resistant property and/or low-temperature storage-resistant property.

19. The method for selecting a yeast according to Claim 18, comprising: culturing a reference yeast and test yeasts; measuring for each yeast the expression level of the gene having the nucleotide sequence of SEQ ID NO: 1 or SEQ ID NO: 3 and encoding a glycogen synthesis initiator; and selecting a test yeast having the gene expression higher than that in the reference yeast.

20. The method for selecting a yeast according to Claim 18, comprising: culturing a reference yeast and test yeasts; quantifying the protein according to Claim 6 in each yeast; and selecting a test yeast having a larger amount of the protein than that in the reference yeast.

## Patentansprüche

1. Polynucleotid, das ausgewählt ist aus der Gruppe bestehend aus:
(a) einem Polynucleotid, das ein Polynucleotid umfasst, das aus der Nucleotidsequenz von SEQ ID NO:1 oder SEQ ID NO:3 besteht;
(b) einem Polynucleotid, das ein Polynucleotid umfasst, das ein Protein codiert, das aus der Aminosäuresequenz von SEQ ID NO:2 oder SEQ ID NO:4 besteht;
(c) einem Polynucleotid, das ein Polynucleotid umfasst, das ein Protein codiert, das aus der Aminosäuresequenz von SEQ ID NO:2 oder SEQ ID NO:4 besteht, in dem eine bis 10 Aminosäuren davon deletiert, substituiert, inseriert und/oder hinzugefügt sind und das eine Glycogen-Synthese-Initiierungsaktivität hat; und
(d) einem Polynucleotid, das ein Polynucleotid umfasst, das ein Protein codiert, das eine Aminosäuresequenz hat, die 90% oder mehr Identität mit der Aminosäuresequenz von SEQ ID NO:2 oder SEQ ID NO:4 hat, und wobei das Protein eine Glycogen-Synthese-Initiierungsaktivität hat.

2. Polynucleotid nach Anspruch 1, das ausgewählt ist aus der Gruppe bestehend aus:
(g) einem Polynucleotid, das ein Polynucleotid umfasst, das ein Protein codiert, das aus der Aminosäuresequenz von SEQ ID NO:2 oder SEQ ID NO:4 besteht oder das die Aminosäuresequenz von SEQ ID NO:2 oder SEQ ID NO:4 codiert, in dem 1 bis 2 Aminosäuren davon deletiert, substituiert, inseriert und/oder hinzugefügt sind und das eine Glycogen-Synthese-Initiierungsaktivität hat; und
(h) einem Polynucleotid, das ein Polynucleotid umfasst, das ein Protein codiert, das 98% oder mehr Identität mit der Aminosäuresequenz von SEQ ID NO:2 oder SEQ ID NO:4 hat, und das eine Glycogen-Synthese-Initiierungsaktivität hat.

3. Polynucleotid nach Anspruch 1, das ein Polynucleotid umfasst, das aus der Nucleotidsequenz von SEQ ID NO:1 oder SEQ ID NO:3 besteht.

4. Polynucleotid nach Anspruch 1, das ein Polynucleotid umfasst, das ein Protein codiert, das aus der Aminosäuresequenz von SEQ ID NO:2 oder SEQ ID NO:4 besteht.

5. Polynucleotid nach einem der Ansprüche 1 bis 4, wobei das Polynucleotid DNA ist.

6. Protein, das von dem Polynucleotid nach einem der Ansprüche 1 bis 5 codiert wird.

7. Vektor, der das Polynucleotid nach einem der Ansprüche 1 bis 5 enthält.

8. Hefe, in die der Vektor nach Anspruch 7 eingeführt ist.

9. Hefe nach Anspruch 8, wobei die trockenresistente Eigenschaft erhöht ist.

10. Hefe nach Anspruch 8, wobei die Niedrigtemperaturlagerung-resistente Eigenschaft erhöht ist.

11. Hefe nach Anspruch 9, wobei die trockenresistente Eigenschaft durch Erhöhen des Expressionsspiegels des Proteins nach Anspruch 6 erhöht ist.

12. Hefe nach Anspruch 10, wobei die Niedrigtemperaturlagerung-resistente Eigenschaft durch Erhöhen des Expressionsspiegels des Proteins nach Anspruch 6 erhöht ist.

13. Verfahren zur Herstellung eines alkoholischen Getränks durch Verwendung der Hefe nach einem der Ansprüche 8 bis 12.

14. Verfahren nach Anspruch 13, wobei das gebraute alkoholische Getränk ein Malzgetränk ist.

15. Verfahren nach Anspruch 13, wobei das gebraute alkoholische Getränk Wein ist.

16. Verfahren zum Beurteilen einer Testhefe auf ihre trockenresistente Eigenschaft und/oder ihre Niedrigtemperaturlagerung-resistente Eigenschaft, umfassend die Verwendung eines Primers oder einer Sonde, der/die basierend auf der Nucleotidsequenz eines Gens konzipiert ist, das die Nucleotidsequenz von SEQ ID NO:1 oder SEQ ID NO:3 hat und das einen Glycogen-Synthese-Initiator codiert.

17. Verfahren zum Beurteilen einer Testhefe auf ihre trockenresistente Eigenschaft und/oder ihre Niedrigtemperaturlagerung-resistente Eigenschaft, umfassend:
Züchten der Testhefe und Messen des Expressionsspiegels des Gens, das die Nucleotidsequenz von SEQ ID NO:1 oder SEQ ID NO:3 hat und das einen Glycogen-Synthese-Initiator codiert.

18. Verfahren für das Auswählen einer Hefe, umfassend: Züchten von Testhefen; Quantifizieren des Proteins nach Anspruch 6 oder Messen des Expressionsspiegels des Gens, das die Nucleotidsequenz von SEQ ID NO:1 oder SEQ ID NO:3 hat und das einen Glycogen-Synthese-Initiator codiert; und Auswählen einer Testhefe, die eine Menge des Protein- oder Genexpressionsspiegels hat, der einer günstigen trockenresistenten Eigenschaft und/oder einer günstigen Niedrigtemperaturlagerung-resistenten Eigenschaft entspricht.

19. Verfahren für das Auswählen einer Hefe nach Anspruch 18, umfassend:
Züchten einer Referenzhefe und von Testhefen; Messen des Expressionsspiegels, für jede Hefe, des Gens, das die Nucleotidsequenz von SEQ ID NO:1 oder SEQ ID NO:3 hat und das einen Glycogen-Synthese-Initiator codiert; und Auswählen einer Testhefe, die die Genexpression hat, die höher ist als die in der Referenzhefe.

20. Verfahren für das Auswählen einer Hefe nach Anspruch 18, umfassend:
Züchten einer Referenzhefe und von Testhefen; Quantifizieren des Proteins nach Anspruch 6 in jeder Hefe; und Auswählen einer Testhefe, die eine größere Proteinmenge hat als die in der Referenzhefe.

## Revendications

1. Polynucléotide choisi dans le groupe constitué de:
(a) un polynucléotide comprenant un polynucléotide consistant en la séquence nucléotidique de SEQ ID NO: 1 ou SEQ ID NO: 3;
(b) un polynucléotide comprenant un polynucléotide codant pour une protéine consistant en la séquence d'acides aminés de SEQ ID NO: 2 ou SEQ ID NO: 4;
(c) un polynucléotide comprenant un polynucléotide codant pour une protéine consistant en la séquence d'acides aminés de SEQ ID NO: 2 ou SEQ ID NO: 4 dans laquelle un à 10 de ses acides aminés sont délétés, substitués, insérés et/ou ajoutés, et ayant une activité d'initiation de la synthèse du glycogène; et
(d) un polynucléotide comprenant un polynucléotide codant pour une protéine ayant une séquence d'acides aminés présentant 90 % ou plus d'identité avec la séquence d'acides aminés de SEQ ID NO: 2 ou SEQ ID NO: 4, et ladite protéine ayant une activité d'initiation de la synthèse du glycogène.

2. Polynucléotide selon la revendication 1 choisi dans le groupe constitué de:
(g) un polynucléotide comprenant un polynucléotide codant pour une protéine consistant en la séquence d'acides aminés de SEQ ID NO: 2 ou SEQ ID NO: 4, ou codant pour la séquence d'acides aminés de SEQ ID NO: 2 ou SEQ ID NO: 4 dans laquelle 1 à 2 de ses acides aminés sont délétés, substitués, insérés, et/ou ajoutés, et dans lequel ladite protéine a une activité d'initiation de la synthèse du glycogène; et
(h) un polynucléotide comprenant un polynucléotide codant pour une protéine présentant 98 % ou plus d'identité avec la séquence d'acides aminés de SEQ ID NO: 2 ou SEQ ID NO: 4, et ayant une activité d'initiation de la synthèse du glycogène.

3. Polynucléotide selon la revendication 1 comprenant un polynucléotide consistant en la séquence nucléotidique de SEQ ID NO: 1 ou SEQ ID NO: 3.

4. Polynucléotide selon la revendication 1 comprenant un polynucléotide codant pour une protéine consistant en la séquence d'acides aminés de SEQ ID NO: 2 ou SEQ ID NO: 4.

5. Polynucléotide selon l'une quelconque des revendications 1 à 4, dans lequel le polynucléotide est de l'ADN.

6. Protéine codée par le polynucléotide selon l'une quelconque des revendications 1 à 5.

7. Vecteur contenant le polynucléotide selon l'une quelconque des revendications 1 à 5.

8. Levure dans laquelle le vecteur selon la revendication 7 a été introduit.

9. Levure selon la revendication 8, dans laquelle la propriété de résistance à la sécheresse est augmentée.

10. Levure selon la revendication 8, dans laquelle la propriété de résistance à un stockage à basse température est augmentée.

11. Levure selon la revendication 9, dans laquelle la propriété de résistance à la sécheresse est augmentée en augmentant le taux d'expression de la protéine selon la revendication 6.

12. Levure selon la revendication 10, dans laquelle la propriété de résistance à un stockage à basse température est augmentée en augmentant le taux d'expression de la protéine selon la revendication 6.

13. Méthode pour la production d'une boisson alcoolisée en utilisant la levure selon l'une quelconque des revendications 8 à 12.

14. Méthode selon la revendication 13, dans laquelle la boisson alcoolisée brassée est une boisson à base de malt.

15. Méthode selon la revendication 13, dans laquelle la boisson alcoolisée brassée est du vin.

16. Méthode pour l'estimation d'une levure à tester pour sa propriété de résistance à la sécheresse et/ou sa propriété de résistance à un stockage à basse température, comprenant l'utilisation d'une amorce ou d'une sonde conçue en se basant sur la
séquence nucléotidique d'un gène ayant la séquence nucléotidique de SEQ ID NO: 1 ou SEQ ID NO: 3 et codant pour un initiateur de la synthèse du glycogène.

17. Méthode pour l'estimation d'une levure à tester pour sa propriété de résistance à la sécheresse et/ou sa propriété de résistance à un stockage à basse température, comprenant: la culture de la levure à tester; et la mesure du taux d'expression du gène ayant la séquence nucléotidique de SEQ ID NO: 1 ou SEQ ID NO: 3 et codant pour un initiateur de la synthèse du glycogène.

18. Méthode pour la sélection d'une levure, comprenant: la culture de levures à tester; la quantification de la protéine selon la revendication 6 ou la mesure du taux d'expression du gène ayant la séquence nucléotidique de la SEQ ID NO: 1 ou de la SEQ ID NO: 3 et codant pour un initiateur de la synthèse du glycogène; et la sélection d'une levure à tester ayant une quantité de protéine ou un taux d'expression du gène en accord avec une propriété de résistance à la sécheresse et/ou une propriété de résistance à un stockage à basse température favorable.

19. Méthode pour la sélection d'une levure selon la revendication 18, comprenant: la culture d'une levure de référence et de levures à tester ; la mesure pour chaque levure du taux d'expression du gène ayant la séquence nucléotidique de SEQ ID NO: 1 ou SEQ ID NO: 3 et codant pour un initiateur de la synthèse du glycogène; et la sélection d'une levure à tester présentant une expression du gène supérieure à celle de la levure de référence.

20. Méthode pour la sélection d'une levure selon la revendication 18, comprenant: la culture d'une levure de référence et de levures à tester; la quantification de la protéine selon la revendication 6 dans chaque levure; et la sélection d'une levure à tester comportant une quantité supérieure de la protéine par rapport à celle dans la levure de référence.


<110> Suntory Limited
<120> Gene encoding glycogen synthesis initiator and use thereof
<130> PCT06-0149
<150> JP2006-55570
   <151> 2006-03-01
<160> 8
<170> PatentIn version 3.3
<210> 1
   <211> 1680
   <212> DNA
   <213> Saccharomyces sp.
<400> 1
   atgtgtagaa aactagccat tgtcacacta ctgtactccg cggattattt accgggcgtg      60
   tttgctcttg gtcatcaagt taacaaactg ttaaaggaag cgggtaggaa agatagaatt     120
   gatgcatgcc ttgttgtgac aactccctta ttcaatgaca ttttgagcga tttggccaaa     180
   gatcttttga aaacaatata cgacgatatc gtacttgtaa accctttgga atgccaagat    240
   gaaagtattc agagaaacag tgaaaatcta gctcttttgg aaaggcccga attgtcgttt     300
   gctctaataa aggcaagact atgggaacta acccagtacg aacaagttct atatttggat     360
   tcagacactt tacccctgaa aaaagacttt ctaagattgt tcgacattat gtctaaacaa    420
   actaagttac agattggtgc tgttgctgac attggctggc cagatatttt caatagcggc     480
   gttatgatgc tgataccaga tgctgatact gcatctgttt tacagaacta tgttatcgaa     540
   aatacttcaa ttgatggtgc tgatcagggc attttgaatc agttttttaa ccaaaactgc     600
   tgcacggatg agctgctcaa agaaagcttt ccccgagagt gggtacagtt atcatttaca     660
   tataatgtga ccacccctaa tcttggttat gaatcttcac ctgctatgaa ttattttaaa     720
   ccaactatca aactgattca tttcattggc caacataaac catggtctct gtggtctcag     780
   acaaactttg ttagaaacga atacaatacc caatggaatg gagtgtacga ggactttaag     840
   caggaaaaca aattggtaga tgacgtctcg aagattgata tcagtgattt taatgaggac     900
   aataatgttc aaacagcatc tcaagaaact attccgcaaa cagcatcttc aggggctatt     960
   cctcaagata atgatttttc tactgaaaag gaagtcgaaa caatagacac aaagcaagaa    1020
   gacaccagag tccagctcaa taagccagct cccgttcctg ttccactgaa tttcactgaa    1080
   tggttgacaa ctttcataaa caaggataat gtgaattccc aggcaatgaa caaaatgcac 1140
   gaacacgaag gaaatgatag tggttttgat aaagacgacg ctaaatcaga tgaagacatc 1200
   tacgtgaata acagcgacgc taaaccagac caagaaagta ttgcggataa aaacgacgct 1260
   aaatcagatc aagacggcca tgtgaataac aatgactcta atccagacca agagagtcac 1320
   gcggatgtca cacaagaccc tattacttat aaagatgaca tatcagagga tatagaaccg 1380
   cctgtgccta ccgaagacga cgtgaaactt ctggaacagg atgaggaggg ttacgatgaa 1440
   ttccttctag acgtatgcga tgcagatgcg atcaacaacg aagaaggaga agatttcgat 1500
   gtaggaaaag tgggaaggag tgttgaagat gcactcgaaa aagagaatcc gacagaggac 1560
   gaacccaaaa attcacctca agaaatgccg aacttcaggt tcgactggga agattctgat 1620
   tacctatcaa aagttgaaag gtatttccct gatgacgtct ttgaatatgc agtagaatga 1680
<210> 2
   <211> 559
   <212> PRT
   <213> Saccharomyces sp.
<400> 2
   Met Cys Arg Lys Leu Ala Ile Val Thr Leu Leu Tyr Ser Ala Asp Tyr
   1               5                   10                  15
   Leu Pro Gly Val Phe Ala Leu Gly His Gln Val Asn Lys Leu Leu Lys
               20                  25                  30
   Glu Ala Gly Arg Lys Asp Arg Ile Asp Ala Cys Leu Val Val Thr Thr
           35                  40                  45
   Pro Leu Phe Asn Asp Ile Leu Ser Asp Leu Ala Lys Asp Leu Leu Lys
       50                  55                  60
   Thr Ile Tyr Asp Asp Ile Val Leu Val Asn Pro Leu Glu Cys Gln Asp
   65                  70                  75                  80
   Glu Ser Ile Gln Arg Asn Ser Glu Asn Leu Ala Leu Leu Glu Arg Pro
                   85                  90                  95
   Glu Leu Ser Phe Ala Leu Ile Lys Ala Arg Leu Trp Glu Leu Thr Gln
               100                 105                 110
   Tyr Glu Gln Val Leu Tyr Leu Asp Ser Asp Thr Leu Pro Leu Lys Lys
           115                 120                 125
   Asp Phe Leu Arg Leu Phe Asp Ile Met Ser Lys Gln Thr Lys Leu Gln
       130                 135                 140
   Ile Gly Ala Val Ala Asp Ile Gly Trp Pro Asp Ile Phe Asn Ser Gly
   145                 150                 155                 160
   Val Met Met Leu Ile Pro Asp Ala Asp Thr Ala Ser Val Leu Gln Asn
                   165                 170                 175
   Tyr Val Ile Glu Asn Thr Ser Ile Asp Gly Ala Asp Gln Gly Ile Leu
   180 185 190
   Asn Gln Phe Phe Asn Gln Asn Cys Cys Thr Asp Glu Leu Leu Lys Glu
   195 200 205
   Ser Phe Pro Arg Glu Trp Val Gln Leu Ser Phe Thr Tyr Asn Val Thr
   210 215 220
   Thr Pro Asn Leu Gly Tyr Glu Ser Ser Pro Ala Met Asn Tyr Phe Lys
   225 230 235 240
   Pro Thr Ile Lys Leu Ile His Phe Ile Gly Gln His Lys Pro Trp Ser
   245 250 255
   Leu Trp Ser Gln Thr Asn Phe Val Arg Asn Glu Tyr Asn Thr Gln Trp
   260 265 270
   Asn Gly Val Tyr Glu Asp Phe Lys Gln Glu Asn Lys Leu Val Asp Asp
   275 280 285
   Val Ser Lys Ile Asp Ile Ser Asp Phe Asn Glu Asp Asn Asn Val Gln
   290 295 300
   Thr Ala Ser Gln Glu Thr Ile Pro Gln Thr Ala Ser Ser Gly Ala Ile
   305 310 315 320
   Pro Gln Asp Asn Asp Phe Ser Thr Glu Lys Glu Val Glu Thr Ile Asp
   325 330 335
   Thr Lys Gln Glu Asp Thr Arg Val Gln Leu Asn Lys Pro Ala Pro Val
   340 345 350
   Pro Val Pro Leu Asn Phe Thr Glu Trp Leu Thr Thr Phe Ile Asn Lys
   355 360 365
   Asp Asn Val Asn Ser Gln Ala Met Asn Lys Met His Glu His Glu Gly
   370 375 380
   Asn Asp Ser Gly Phe Asp Lys Asp Asp Ala Lys Ser Asp Glu Asp Ile
   385 390 395 400
   Tyr Val Asn Asn Ser Asp Ala Lys Pro Asp Gln Glu Ser Ile Ala Asp
   405 410 415
   Lys Asn Asp Ala Lys Ser Asp Gln Asp Gly His Val Asn Asn Asn Asp
   420 425 430
   Ser Asn Pro Asp Gln Glu Ser His Ala Asp Val Thr Gln Asp Pro Ile
   435 440 445
   Thr Tyr Lys Asp Asp Ile Ser Glu Asp Ile Glu Pro Pro Val Pro Thr
   450 455 460
   Glu Asp Asp Val Lys Leu Leu Glu Gln Asp Glu Glu Gly Tyr Asp Glu
   465 470 475 480
   Phe Leu Leu Asp Val Cys Asp Ala Asp Ala Ile Asn Asn Glu Glu Gly
                   485                 490                 495
   Glu Asp Phe Asp Val Gly Lys Val Gly Arg Ser Val Glu Asp Ala Leu
               500                 505                 510
   Glu Lys Glu Asn Pro Thr Glu Asp Glu Pro Lys Asn Ser Pro Gln Glu
           515                 520                 525
   Met Pro Asn Phe Arg Phe Asp Trp Glu Asp Ser Asp Tyr Leu Ser Lys
       530                 535                 540
   Val Glu Arg Tyr Phe Pro Asp Asp Val Phe Glu Tyr Ala Val Glu
   545                 550                 555
<210> 3
   <211> 1143
   <212> DNA
   <213> Saccharomyces sp.
<400> 3
   atgattaaga aagttgctct ttgcacgtta ttatattcac gggggtattt acctggtgcc      60
   ttaactttag cgtaccaatt acaaggactc ttgagccaga ctgtagtaga ggacgaaata     120
   actatatgtc tattagttgc aagaacgttg ttcgaagatg aatttactcc ccaggaaata     180
   gttttgataa gaagcctctt caaggagatc attatcatag aatcgttaga gggtcaagcg     240
   aagagcgtcg aaaataacaa ggcaaatctt gacttgttga aaaggccgga gctatctttt     300
   actttactaa aagccagatt atgggaactg gtgcagttcg atcaggtgct gtttttggat     360
   gcggatactt taccattgaa caaggagttt tttaaaattc tacagctata tcctgaacaa     420
   acgaggttcc agatcgctgc tgttcccgat atcggatggc ccgatatgtt taacaccggt     480
   gtcttgctac tgattccaga tttggaaatg gcgaagagct tgcaggattt tttggtcaag     540
   actgtatcga ttgacggggc tgatcaaggt attttcaatc aatttttcaa ccccgcatgc     600
   aattacagca aggagatctt gcacaaaata tctccgctta tggagtggat acgacttccg     660
   ttcatctata acgtaacaat gcccaattac ggataccagt cctcgcctgc gatgagcttt     720
   ttccagcagc atatcaacct agttcatttc atcggaacgt tcaaaccgtg gtcacatagt     780
   gcattcaacc acgataatta ttattatcaa ttatggaggg atacagagag ggatctacac     840
   aacgaacacc acttgagtaa ttatttcaca cgcttgcaac tcggcaatct cgaaagggaa     900
   acagatttcc atcacgaggc tccctgcatc aacacgctgc taaggcaaaa ctcaagggag     960
   aatcaaaagc aggttagccc tgatgaaacc caggtaggtc ctaatgctgc acaaaatata    1020
   cctatacaag aacacgatca aaaagtgaca actgctgata ctcaaagtgc cttcaagttt    1080
   gagtgggaga atacggacta tttgcaccac gtccagagag cgttcccaga ctccgatatc    1140
   taa                                                                  1143
<210> 4
   <211> 380
   <212> PRT
   <213> Saccharomyces sp.
<400> 4
   Met Ile Lys Lys Val Ala Leu Cys Thr Leu Leu Tyr Ser Arg Gly Tyr
   1 5 10 15
   Leu Pro Gly Ala Leu Thr Leu Ala Tyr Gln Leu Gln Gly Leu Leu Ser
   20 25 30
   Gln Thr Val Val Glu Asp Glu Ile Thr Ile Cys Leu Leu Val Ala Arg
   35 40 45
   Thr Leu Phe Glu Asp Glu Phe Thr Pro Gln Glu Ile Val Leu Ile Arg
   50 55 60
   Ser Leu Phe Lys Glu Ile Ile Ile Ile Glu Ser Leu Glu Gly Gln Ala
   65 70 75 80
   Lys Ser Val Glu Asn Asn Lys Ala Asn Leu Asp Leu Leu Lys Arg Pro
   85 90 95
   Glu Leu Ser Phe Thr Leu Leu Lys Ala Arg Leu Trp Glu Leu Val Gln
   100 105 110
   Phe Asp Gln Val Leu Phe Leu Asp Ala Asp Thr Leu Pro Leu Asn Lys
   115 120 125
   Glu Phe Phe Lys Ile Leu Gln Leu Tyr Pro Glu Gln Thr Arg Phe Gln
   130 135 140
   Ile Ala Ala Val Pro Asp Ile Gly Trp Pro Asp Met Phe Asn Thr Gly
   145 150 155 160
   Val Leu Leu Leu Ile Pro Asp Leu Glu Met Ala Lys Ser Leu Gln Asp
   165 170 175
   Phe Leu Val Lys Thr Val Ser Ile Asp Gly Ala Asp Gln Gly Ile Phe
   180 185 190
   Asn Gln Phe Phe Asn Pro Ala Cys Asn Tyr Ser Lys Glu Ile Leu His
   195 200 205
   Lys Ile Ser Pro Leu Met Glu Trp Ile Arg Leu Pro Phe Ile Tyr Asn
   210 215 220
   Val Thr Met Pro Asn Tyr Gly Tyr Gln Ser Ser Pro Ala Met Ser Phe
   225 230 235 240
   Phe Gln Gln His Ile Asn Leu Val His Phe Ile Gly Thr Phe Lys Pro
   245 250 255
   Trp Ser His Ser Ala Phe Asn His Asp Asn Tyr Tyr Tyr Gln Leu Trp
   260 265 270
   Arg Asp Thr Glu Arg Asp Leu His Asn Glu His His Leu Ser Asn Tyr
           275                 280                 285
   Phe Thr Arg Leu Gln Leu Gly Asn Leu Glu Arg Glu Thr Asp Phe His
       290                 295                 300
   His Glu Ala Pro Cys Ile Asn Thr Leu Leu Arg Gln Asn Ser Arg Glu
   305                 310                 315                 320
   Asn Gln Lys Gln Val Ser Pro Asp Glu Thr Gln Val Gly Pro Asn Ala
                   325                 330                 335
   Ala Gln Asn Ile Pro Ile Gln Glu His Asp Gln Lys Val Thr Thr Ala
               340                 345                 350
   Asp Thr Gln Ser Ala Phe Lys Phe Glu Trp Glu Asn Thr Asp Tyr Leu
           355                 360                 365
   His His Val Gln Arg Ala Phe Pro Asp Ser Asp Ile
       370                 375                 380
<210> 5
   <211> 40
   <212> DNA
   <213> Artificial
<220>
   <223> Primer
<400> 5
   gagctcatag cggccatgtg tagaaaacta gccattgtca 40
<210> 6
   <211> 42
   <212> DNA
   <213> Artificial
<220>
   <223> Primer
<400> 6
   ggatcctatg cggccgcgta cgtgaaagaa ctacactttc tt 42
<210> 7
   <211> 40
   <212> DNA
   <213> Artificial
<220>
   <223> Primer
<400> 7
   gagctcatag cggccatgat taagaaagtt gctctttgca 40
<210> 8
   <211> 42
   <212> DNA
   <213> Artificial
<220>
   <223> Primer
<400> 8
   ggatcctatg cggccgccag cgaattgacc cggccttact at 42